Europäisches Patentamt

European Patent Office

Office européen des brevets

(11) Numéro de publication: **0 273 800 B1**

# FASCICULE DE BREVET EUROPEEN

(45) Date de publication de fascicule du brevet: **11.03.92**

(51) Int. Cl.5: **C07K 15/00**, C12P 21/02, A61K 37/02

(21) Numéro de dépôt: **87402696.6**

(22) Date de dépôt: **30.11.87**

(54) **Variants de l'hirudine, leur utilisation et leur préparation.**

(30) Priorité: **01.12.86 FR 8616723**

(43) Date de publication de la demande:
**06.07.88 Bulletin 88/27**

(45) Mention de la délivrance du brevet:
**11.03.92 Bulletin 92/11**

(84) Etats contractants désignés:
**AT BE CH DE ES FR GB GR IT LI LU NL SE**

(56) Documents cités:
**EP-A- 0 158 564
EP-A- 0 168 342
EP-A- 0 171 024
EP-A- 0 200 655
WO-A-86/03517**

**PROCEEDINGS OF THE NATIONAL ACADEMY
OF SCIENCES OF THE USA, vol. 83, no. 4,
février 1986, pages 1084-1088, Medical Sciences, Washington, US; R.P. HARVEY et al.:
"Cloning and expression of a cDNA coding
for the anticoagulant hirudin from the bloodsucking leech, Hirudo medicinalis"**

(73) Titulaire: **TRANSGENE S.A.
11, rue de Molsheim
F-67082 STRASBOURG CEDEX(FR)**

(72) Inventeur: **Courtney, Michael
11 rue des Lilas
F-67170 Geudertheim(FR)**
Inventeur: **Degryse, Eric
6 rue d'Oslo
F-67000 Strasbourg(FR)**
Inventeur: **Loison, Gérard
19 rue du Faubourg National
F-67000 Strasbourg(FR)**
Inventeur: **Lemoine, Yves
4 rue des Alisiers
F-67100 Strasbourg-Neudorf(FR)**

(74) Mandataire: **Warcoin, Jacques et al
Cabinet Régimbeau 26, avenue Kléber
F-75116 Paris(FR)**

**Description**

Au moins 3 variants naturels de l'hirudine sont décrits dans la littérature (Markwardt 1970 ; Petersen et al. 1976 ; Markwardt et Walsmann 1976 ; Chang 1983 ; Dodt et al. 1984, 1985, 1986 ; Harvey et al. 1986 ; brevet français 84.04755).

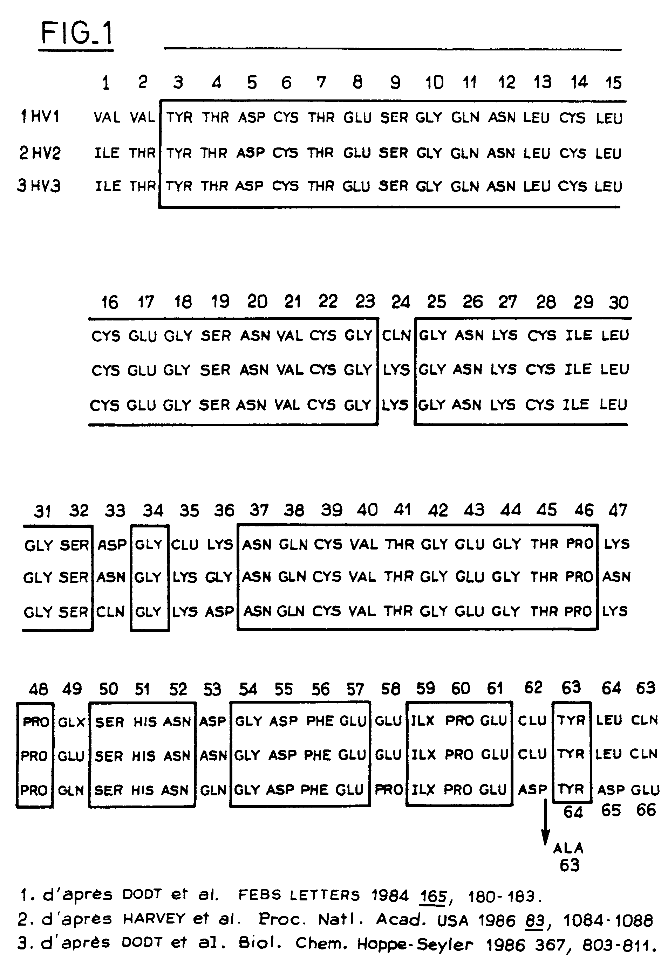

Les séquences comparées de ces 3 variants sont présentées dans la figure 1.

Le premier variant, HV1, correspond à l'hirudine qu'on isole du corps de la sangsue ; le deuxième, HV2 (Harvey et al. 1986) diffère du premier par 9 acides aminés ; le troisième (Dodt et al. 1986) est identique à HV2 jusqu'à la sérine 32 mais diffère par 10 acides aminés dans la partie C-terminale, qui comprend, en outre, un acide aminé supplémentaire (ala63). Ce troisième variant sera désigné ci-après HV3.

Ces séquences comportent 65 ou 66 acides aminés et peuvent être considérées comme 2 domaines : une partie N-terminal globulaire qui contient 3 ponts disulfures et une partie C-terminale acide qui présente une homologie avec le site de clivage par la thrombine dans la molécule de prothrombine. Cette homologie permet de penser que la région qui entoure la position 47 pourrait être le site de fixation de l'hirudine sur la thrombine.

D'autre part, l'hirudine naturelle contient une tyrosine sulfatée, en position 63 (Chang, 1983). Cette tyrosine sulfatée se retrouve en position 64 dans le variant HV3. Elle sera désignée ci-après comme "position 63" pour tous les variants, sa fonction étant la même quelle que soit sa position.

L'analyse comparée des séquences des variants naturels de l'hirudine permet d'envisager, théoriquement, la création de nouveaux variants où sont combinées différemment les caractéristiques des molécules naturelles.

HV1 et HV3 présentent une lysine en position 47, située entre 2 prolines, qui est probablement responsable du blocage du site actif de la thrombine (Dodt et al. 1984 et 1985).

Comme HV2 ne présente pas de résidu basique à cette position mais une asparagine, il parait souhaitable d'y substituer une lysine ou une arginine pour la rendre plus conforme à ce qu'on attend d'un inhibiteur de la thrombine (Chang 1985) ou encore une histidine qui n'est pas conforme pour un bon substrat de la thrombine mais qui pourrait augmenter le pouvoir inhibiteur de l'hirudine.

D'autre part la sulfatation de la tyrosine 63 constitue une différence entre l'hirudine naturelle et l'hirudine obtenue par recombinaison génétique qui pourrait avoir des conséquences sur son activité, comme le suggèrent les études cinétiques de Stone et Hofsteenge (1986).

On peut tenter de mimer la protéine native en remplaçant la tyrosine par un résidu acide comme glu ou asp.

La présente invention concerne des variants de l'hirudine, caractérisés en ce qu'ils comportent un acide aminé différent de l'acide aminé existant dans la forme naturelle en position 47 ou 63.

Il s'agit de préférence d'un variant de l'hirudine HV1, HV2 ou HV3.

Parmi les variants en cause, il faut citer :

- les variants dans lesquels l'acide aminé $Tyr^{63}$ a été remplacé par un résidu acide, en particulier Glu ou Asp ;
- les variants dans lesquels l'acide aminé en position 47 dans la forme naturelle est remplacé par une Arg ou His ;
- les variants dans lesquels l'$Asn^{47}$ de la forme naturelle de HV2 est remplacé par une Lys.

Il faut en particulier, citer les variants préférés suivants :

$[Lys^{47}]$ HV2

$[Arg^{47}]$ HV2

$[His^{47}]$ HV2

$[Glu^{63}]$ HV2

$[Asp^{63}]$ HV2

Le variant préférré de la présente invention, à savoir $(Lys^{47})$HV2 [ou "rHV2-Lys 47"], répond à la formule suivante :

```
ILE THR TYR THR ASP CYS THR GLU SER GLY GLN ASN LEU CYS LEU
CYS GLU GLY SER ASN VAL CYS GLY LYS GLY ASN LYS CYS ILE LEU
GLY SER ASN GLY LYS GLY ASN GLN CYS VAL THR GLY GLU GLY THR
PRO LYS PRO GLU SER HIS ASN ASN GLY ASP PHE GLU GLU ILE PRO
GLU GLU TYR LEU GLN
```

Les différents variants qui ont conservé la Tyr[63] peuvent être utilisés sous forme sulfatée ou non. Cette sulfatation peut être obtenue chimiquement ou bien par voie biologique.

La présente invention s'étend aux procédés biologiques et/ou chimiques permettant de préparer les variants mentionnés ci-dessus.

On peut en effet obtenir ces variants par synthèse, hémisynthèse et/ou par les techniques connues de manipulation génétique.

Ainsi, par exemple, le clonage et l'expression des différentes séquences codant pour HV1, HV2 et HV3, en particulier HV2, et l'obtention des hirudines correspondantes à partir de cultures de levures ont été décrits dans la demande de brevet EP-A-200 655.

Les variants selon l'invention peuvent être obtenus par des techniques équivalentes après avoir modifié les séquences codantes mentionnées précédemment, par exemple par mutagénèse dirigée.

En particulier, par mutagénèse dirigée in vitro, on a construit des variants où l'asparagine 47 est remplacée par une lysine, une arginine ou une histidine et où la tyrosine 63 est remplacée par un acide glutamique.

En particulier, il est possible d'utiliser des blocs d'expression fonctionnels tels que décrits dans la demande de brevet EP-A-200 655 et dans lesquels la séquence de l'hirudine code pour les variants précédents ; ces blocs fonctionnels d'ADN peuvent être portés par un plasmide vecteur.

Pour diriger l'expression et la sécrétion par la levure des gènes correspondant aux différents variants, ceux-ci sont intégrés dans un vecteur de levure qui comprend, de préférence, les éléments suivants, qui ont été décrits dans la demande EP-A-200 655 :

- l'origine de réplication du plasmide de levure 2 μ
- le gène ura3,
- une origine de réplication dans E. coli et un marqueur de résistance à un antibiotique,
- un promoteur de transcription, la séquence "leader" et la séquence pre-pro du précurseur du facteur alpha, fusionnée en phase, en amont de la séquence codante du variant de l'hirudine,
- le terminateur de transcription du gène PGK de la levure, qui sera placé en aval du gène dudit variant.

L'invention concerne également les levures transformées par ces vecteurs ou par ce bloc fonctionnel d'ADN et leur application à la préparation des variants de l'hirudine.

En particulier, l'invention concerne un procédé de préparation d'un variant de l'hirudine par fermentation d'une levure selon l'invention et récupération de l'hirudine produite dans le milieu de culture sous forme mature ou sous forme d'un précurseur maturable in vitro.

Les techniques mises en oeuvre ont déjà été décrites plus en détail dans les demandes de brevet EP-A-200 655 et EP-87 401649.6

Les variants de l'hirudine ainsi obtenus peuvent être utilisés comme cela est décrit dans la demande de brevet EP-A-200 655 en tant qu'inhibiteur de la thrombine, tant in vivo que in vitro.

En particulier, ces variants peuvent être utilisés dans des compositions pharmaceutiques, seuls ou en combinaison avec d'autres principes actifs, ou bien dans le cadre de tests ou de diagnostic, in vitro ou in vivo. Dans ce dernier cas, il peut être intéressant de marquer les variants, par exemple par un marquage radioactif, fluorescent, enzymatique ou autre.

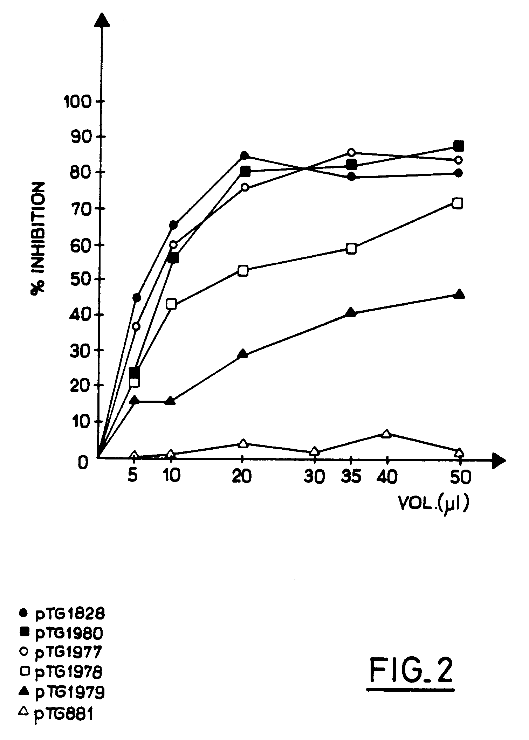

La présente invention est illustrée par les exemples suivants à l'aide de la figure 1 qui représente les séquences de 3 variants naturels de l'hirudine, à savoir HV1, HV2 et HV3 et à l'aide de la figure 2 qui représente le pourcentage d'inhibition de l'activité protéolytique de la thrombine sur le chromozyme en fonction des volumes de surnageants des cultures de levure produisant l'hirudine rHV$_2$ ou ses variants.

Exemple I Constructions des différents variants de HV-2 par mutagénèse in vitro.

Pour effectuer une mutagénèse dirigée in vitro, le fragment d'ADN que l'on veut modifier est cloné dans la forme réplicative d'un phage simple brin ; le génome du phage recombinant est isolé et mis à hybrider avec un oligonucléotide synthétique qui porte la séquence mutée. Cet oligonucléotide sert d'amorce à la synthèse du brin complémentaire et l'ADN rendu ainsi double-brin est utilisé pour transformer une bactérie réceptrice qui va produire du phage portant la mutation recherchée (Zoller and Smith, 1983).

Le plasmide pTG720, décrit dans le brevet EP-A-158564 est un vecteur d'expression de l'hirudine dans E. coli. Le plasmide pTG730 a été dérivé de pTG720 par l'adjonction d'un site EcoRI en aval de la séquence codante de l'hirudine. Le site EcoRI permet de récupérer la séquence codante de l'hirudine par digestion EcoRI d'une part, ClaI d'autre part.

Le fragment ClaI-EcoRI couvre la totalité de la région codante de l'hirudine moins quelques codons 5'-terminaux. Ce fragment ClaI-EcoRI a été cloné entre les sites AccI et EcoRI d'un dérivé du phage M13, appelé M13TG131 (Kieny et al., 1983). Le phage dérivé de M13TG131 et qui comprend cette séquence d'hirudine est nommé M13TG1919.

On a synthétisé trois oligonucléotides qui sont chacun capables de s'apparier avec la séquence 5'-GFACACCGAACCCTGAAAG-3' du M13TG1919 sauf pour leur région centrale qui correspond à la mutation désirée. Les séquences de ces oligonucléotides sont les suivantes :

$$\textbf{TG435} \quad \textbf{5'-CTTTCAGG\underline{GTG}CGGTGTAC-3'}$$

$$\textbf{TG436} \quad \textbf{5'-CTTTCAGG\underline{TTT}CGGTGTAC-3'}$$

$$\textbf{TG437} \quad \textbf{5'-CTTTCAGG\underline{GCG}CGGTGTAC-3'}$$

Ces oligonucléotides ont été conçus pour permettre la substitution du codon AAC (Asn) du M13TG1919 par CAC (his) ou AAA (lys) ou CGC (arg) respectivement.

120 picomoles de chaque oligonucléotide ont été phosphorylés dans 100 $\mu$l de milieu réactionnel et environ 20 picomoles d'oligonucléotide phosphorylé ont été hybridés avec 1 picomole d'ADN simple brin du phage M13TG1919 dans 25 microlitres de tampon d'hybridation.

Après hybridation, le mélange d'ADNs a été soumis à l'action de la polymérase de Klenow et à la ligase du phage T4. Chaque mélange ainsi traité a servi à transfecter la souche de E. coli 71/18 (mut L) sur tapis de bactéries indicatrices JM103 (Messing et al., 1981). Les cellules infectées sont repérables parce qu'elles forment des plages de croissance plus lente ; les colonies ont été repiquées sur milieu complet puis transférées sur papier Whatman 540 en vue d'un criblage des cellules présentant les phages mutés.

Ce criblage est réalisé par hybridation "in situ" avec l'oligonucléotide ayant servi précédemment comme force. On a ainsi obtenu trois nouveaux phages présentant la séquence mutée recherchée :

M13TG1921 (asn$^{47}$ → arg), M13TG1924 (asn$^{47}$ → his), et M13TG1925 (asn$^{47}$ → lys).

D'autre part, le même type d'expérience a été mené avec un oligonucléotide TG434 de séquence 5'-ATTGTAACTCTTCTTCTG-3' Cet oligonucléotide s'hybride avec la séquence 5'-CAGAAGAATATTTACAAT localisée dans la région 3' de la séquence codant pour HV-2, avec un triplet non apparié destiné à substituer le codon TAT (tyr$^{63}$) par GAG (glu). On a ainsi obtenu le phage muté correspondant M13TG1922 (tyr$^{63}$ → glu).

Exemple 2 Substitution du fragment PstI-HindIII (0.6 kb) de pTG1828 par le fragment muté.

Le plasmide pTG1828 (décrit dans la demande de brevet EP-87 401649.6) porte la séquence codante de l'hirudine, précédée des séquences pre-pro du gène de la phéromone sexuelle alpha de levure ; l'ensemble est placé sous le contrôle du promoteur PGK. Par cette fusion entre les séquences pre-pro de MF$\alpha$ et hirudine, la protéine exprimée suit le chemin normal de sécrétion et de maturation de la levure de sorte que l'hirudine processée et active se retrouve dans le milieu de culture.

Ce plasmide a été repris pour exprimer les séquences d'hirudine mutée, à la place de l'hirudine native.

La digestion par PstI et HindIII de pTG1828 libère cinq fragments de tailles approximatives de 3.8, 1.9, 1.5, 1.1 et 0.6 kb respectivement. Le dernier fragment comprend la séquence fusionnée pre-pro-hirudine. Ce fragment ne comprend qu'un seul site AccI et un seul site EcoRI. La région délimitée par ces deux sites comprend l'intégralité de la séquence hirudine moins quelques codons 5'-terminaux. Le fragment HindIII-PstI de 0.6 kb a été inséré entre les sites HindIII et PstI d'un vecteur qui ne présente ni site EcoRI, ni site AccI.

Le plasmide ainsi reconstitué (pTG1960) possède donc un site AccI unique et un site EcoRI unique localisés respectivement au début de la séquence hirudine et en aval de celle-ci. La digestion de pTG1960 par AccI et EcoRI libère un fragment qui comprend la quasi totalité de la séquence de l'hirudine et le reste du plasmide.

Le grand fragment d'ADN délété de la séquence de l'hirudine est purifié sur gel et mélangé au produit de digestion AccI-EcoRI de la forme réplicative de chacun des phages mutés décrits dans l'exemple 1, en vue de reconstituer la fusion pre-pro-hirudine avec les nouveaux variants HV-2 obtenus par mutagénèse dirigée. Quatre nouveaux plasmides ont été sélectionnés : pTG1963 (asn$^{47}$ → arg), pTG1964 (tyr$^{63}$ → glu), pTG1965 (asn$^{47}$ → his) et pTG1966 (asn$^{47}$ → lys) qui ne diffèrent de pTG1960 que par les codons mutés.

Ces séquences portant les codons mutés peuvent être récupérées sous forme de fragments PstI-HindIII pour être reclonées dans le vecteur pTG1828 à la place du fragment PstI-HindIII original.

On a ainsi obtenu 4 nouveaux plasmides : pTG1977 (asn$^{47}$ → arg), pTG1978 (tyr$^{63}$ → glu), pTG1979 (asn$^{47}$ → his) et pTG1980 (asn$^{47}$ → lys) qui ne diffèrent de pTG1828 décrit plus haut que par les mutations créées in vitro.

Exemple 3 Transformation de TGY1sp4 par les ADNs de pTG1977, pTG1978, pTG1979 et pTG1980.

Les cellules de S. cerevisiae TGY1sp4 (Matα ura3.251.373.382 his3.11.15) ont été transformées par les ADNs de pTG1977, pTG1978, pTG1979 et pTG1980. Des transformants ura$^+$ ont été obtenus dans chaque cas.

On a donc 4 souches TGY1sp4 pTG1977, TGY1sp4 pTG1978, TGY1sp4 pTG1979 et TGY1sp4 pTG1980. On a comparé les productions d'hirudine de ces 4 souches et de TGY1sp4 pTG1828.

On a ensemencé 20 ml de culture (YNBG + casaminoacides 0.5 %) à $DO_{660}$ 0.02 avec chaque souche. Après 48 h d'agitation à 30°C, les cultures sont centrifugées (5000 rpm, 5 min) et les surnageants sont dosés.

Une culture de TGY1sp4 pTG881 (plasmide ne portant pas de séquence codant pour HV-2) est utilisée comme contrôle.

L'activité des surnageants bruts est mesurée par leur effet inhibiteur sur l'activité de la thrombine (activité protéolytique sur un substrat synthétique, le chromozyme TH - Boehringer Mannheim). Le pourcentage d'inhibition en fonction des volumes de surnageant est donné dans la figure 2.

On constate que l'effet inhibiteur produit par les variants arg$^{47}$ et lys$^{47}$ est au moins égal à celui de l'HV2 native. Les variants glu$^{63}$ et his$^{47}$ sont légèrement moins inhibiteurs que HV-2.

Exemple 4 Inhibition de l'activité de la thrombine

Pour mieux mettre en évidence l'intérêt des variants de l'hirudine objets de l'invention, en particulier en vue de leur utilisation pharmaceutique, les exemples comparatifs suivants illustrent les propriétés inhibitrices vis à vis de la thrombine du variant $HV_2$ et de deux autres variants de la forme $HV_2$, où l'acide aminé en position 47 est remplacé par une Arg ou une Lys, tous obtenus par ADN recombinant.

Pour étudier la cinétique d'inhibition de la thrombine par ces variants, on utilise la théorie des inhibiteurs à forte affinité, telle qu'elle est décrite dans WIILIAMS J.W. et MORRISON J.F. Methods in Ezymology 63 p. 437-467, 1979, puisque l'hirudine est un inhibiteur réversible de la thrombine.

Les variants de l'hirudine utilisés sont purifiés à 95 % et la thrombine humaine pure de Sigma présente un pourcentage d'activité mesuré par titration du site actif qui est au delà de 92 %. La concentration de la thrombine humaine est la même pour toutes les mesures, soit $5,5 \ 10^{-9}$ M. Le milieu est un tampon PIPES de sodium 0,05 M, pH 7.9, Kcl 0,18 M et PEG 0,1 % à 37 ° C. Le substrat de la thrombine est le chromozyme PL de Boehringer. On respecte un temps de préincubation de 2 minutes pour la thrombine et l'hirudine, puis la réaction est déclenchée avec le substrat.

Le tableau suivant récapitule les valeurs déterminées expérimentalement de la quantité d'hirudine nécessaire pour inhiber 95 % d'une même quantité de thrombine humaine ($5,5 \ 10^{-9}$ M).

|  | Quantité pour atteindre 95 % d'inhibition de la thrombine ($10^{-9}$ M) |
|---|---|
| $HV_2$ LYS$^{47}$ | 7.6 |
| $HV_2$ Arg$^{47}$ | 8.5 |
| HV 2 | 32,2 |

Il apparaît donc qu'il faut environ 4 fois moins d'hirudine $HV_2$ Arg$^{47}$ et $HV_2$ Lys$^{47}$ pour inhiber une même quantité de thrombine humaine que l'hirudine $HV_2$.

Les variants proposés présentent donc des propriétés inhibitrices de la thrombine qui sont nettement améliorées.

Exemple 5 Etude pharmacologique préliminaire de deux variants d'hirudine recombinante rHV2 et rHV2-Lys 47 comparés à l'heparine standard.

1 - BUT DE L'ETUDE

Evaluer deux variants d'hirudine recombinante rHV2 (ou HV2) et rHV2-Lys 47 (ou (Lys$^{47}$)HV2 et comparer leur efficacité antithrombotique à l'hérapine standard. Ces deux variants sont dépourvus de la sulfatation post-transcriptionnelle du groupement Tyr 63.

2 - CONDITIONS EXPERIMENTALES

5

a) L'activité antithrombinique spécifique (thrombines humaine et bovine) des deux hirudines recombinantes en sérum physiologique est déterminée par l'inhibition de l'activité protéolytique de la thrombine sur le chromozyme.

b) L'activité anticoagulante des deux hirudines est comparée in vitro dans le plasma de rat et de lapin par prolongement du temps de thrombine et l'effet anti-IIa est mesuré par méthode chromogénique.

c) La cinétique de disparition plasmatique de l'hirudine après injection par voie i.v. bolus est suivie par la mesure de l'effet anti-IIa à l'aide du temps de thrombine et par méthode chromogénique au moyen de courbes de calibrages.

d) Les concentrations plasmatiques obtenues après 30 minutes de perfusion continue i.v. chez le lapin sont déterminées par les mêmes techniques.

e) L'activité antithrombotique des deux variants d'hirudine et de l'héparine standard est étudiée dans le modèle de Wessler lapin et rat, avec de la thromboplastine comme agent thrombogénique et dans le modèle de stase chez le lapin (Fareed). Les drogues sont administrées en perfusion continue chez le lapin et en i.v. bolus chez le rat.

Les modèles utilisés sont les suivants :

Modèle de Wessler chez le lapin

Des lapins New Zealand mâles pesant 2,5 à 3 kg ont été utilisés dans cette étude. Après anesthésie par injection intraveineuse de pentobarbital sodique (30 mg/kg), la carotide gauche a été canulée et les deux veines jugulaires ont été isolées ; deux ligatures lâches ont été posées sur chacune d'elles à 2,5 cm l'une de l'autre. Les lapins ont reçu ensuite soit du sérum physiologique soit des solutions d'hirudine ou d'héparine en perfusion continue pendant 30 minutes, sous un débit de 2,5 ml/heure. Deux minutes avant la fin de la perfusion, des prélèvements artériels ont été réalisés afin de déterminer les taux plasmatiques des anticoagulants. Une minute avant la fin de la perfusion, de la thromboplastine humaine (Manchester Comparative Reagents LTD), standardisée selon la procédure de M. Buchanan, a été injectée à la dose de 600 ug/kg pendant exactement 30 secondes dans l'aorte via la carotide. Trente secondes plus tard, la perfusion a été arrêtée et une stase sanguine a été réalisée en serrant les ligatures des deux segments veineux pendant 15 minutes. Les jugulaires ont été ouvertes et les thrombi extraits, rincés dans du sérum physiologique et pesés après tamponnement sur papier filtre.

Modèle de Wessler chez le rat

Des rats Sprague-Dawley (CD-COBS) mâles pesant 300 g environ ont été utilisés. Après anesthésie au pentobarbital sodique par voie i.p. (30 mg/kg) et laparotomie médiane, la veine cave inférieure a été dégagée sur 1 cm à partir du carrefour rénal. Deux ligatures souples ont été mises en place à 0,7 cm de distance.

Les produits à tester, dilués dans le sérum physiologique ont été injectés en i.v. bolus à 1 ml/kg 5 min 40 s ou 1 min (héparine) avant la réalisation de la stase veineuse. Quarante secondes avant cette stase, un agent thrombogène (thromboplastine de lapinE. Dade) a été injecté à la dose de 25 mg/ml/kg dans la veine du pénis en 30 secondes très exactement. Dix secondes après la fin de l'injection, une stase a été établie en serrant les deux ligatures, d'abord la proximale, puis la distale. La stase a été maintenue pendant 10 minutes, puis le thrombus a été prélevé, immergé dans une solution de citrate à 0,38 %, séché sur papier essuie-tout et pesé le lendemain après séchage d'une heure à 50° C.

Modèle de thrombose par stase sanguine chez le lapin

Des lapins New Zealand mâles pesant 2,5 à 3 kg ont été utilisés dans cette étude. Après anesthésie par voie intraveineuse avec du pentobarbital sodique (30 mg/kg), la carotide gauche a été canulée et les deux veines jugulaires ont été isolées ; deux ligatures lâches ont été posées sur chacune d'elles à 2,5 cm l'une de l'autre. Les lapins ont reçu ensuite soit du sérum physiologique soit des solutions d'hirudine ou d'héparine en perfusion continue pendant 30 minutes, sous un débit de 2,5 ml/heure. Deux minutes avant la fin de la perfusion, des prélèvements artériels ont été réalisés afin de déterminer les taux plasmatiques des anticoagulants. En même temps que la perfusion a été arrêtée, une stase a été établie en serrant les quatre ligatures (d'abord la proximale puis la distale). La stase a été maintenue pendant 2 heures, puis les jugulaires ont été ouvertes et les thrombic extraits, rincés dans du sérum physiologique et pesés après tamponnement sur papier filtre.

3 - RESULTATS

a) Pour une préparation de solution-mère à la concentration de 1 mg/ml, l'activité antithrombinique spécifique des deux variants d'hirudine vis à vis des thrombines humaine et bovine est exprimée dans le tableau 1 suivant :

Tableau 1

|  | r HV2 | rHV2-Lys 47 |
|---|---|---|
| pour thrombine humaine | 14 800 | 19 000 |
| pour thrombine bovine | 15 500 | 19 200 |
| (résultats attendus pour la thrombine humaine) | (13 300) | (16 000) |

L'activité antithrombinique spécifique de l'hirudine variant rHV2 est de +/- 15 000 ATU/mg et de +/- 19 000 ATU/mg pour le variant rHV2-Lys 47. Ces activités spécifiques sont supérieures à celles attendues. L'activité spécifique est identique pour les thrombines humaine et bovine.

b) L'activité anticoagulante en plasma de rat et de lapin déterminée par temps de thrombine est équivalente pour les faibles concentrations des deux hirudines. Le variant rHV2-Lys 47 est nettement supérieur pour des concentrations plus élevées. La quantité de thrombine résiduelle dans le plasma de rat et de lapin, déterminée par substrat chromogène est comparable après l'ajout des deux hirudines jusqu'à 60 ATU/ml. Pour neutraliser d'avantage les traces de thrombine résiduelle, le variant rHV2-Lys 47 est plus efficace. Ceci reflète la différence des Ki.

c) La cinétique de disparition plasmatique des deux hirudines après injection par voie i.v. bolus est comparable pour la détermination par méthode chromogénique mais légèrement différente pour la détermination par le temps de thrombine (tableau 2). Les hirudines disparaissent selon deux exponentielles. Une première phase (distribution) avec un $t\ 1/2\ \alpha$ d'environ 3 min qui réduit de 60 % la première quantité initiale en 5 min pour les deux hirudines ; une deuxième phase (élimination) avec un $t\ 1/2\ \beta$ de 16 min pour rHV2-Lys 47 et de 28 min pour rHV2 si l'on considère le temps de thrombine et de 28 et 30 min respectivement si l'on utilise la méthode chromogénique.. L'héparine standard disparait selon une seule phase exponentielle ($t\ 1/2\ =\ 9$ min)

Tableau 2

| Demi-vie en minutes des hirudines après injection IV bolus chez le lapin. | | | | |
|---|---|---|---|---|
| Méthode | $t\ 1/2\ \alpha$ | | $t\ 1/2\ \beta$ | |
|  | TT | SC | TT | SC |
| rHV2 | 2,5 | 3,0 | 28 | 28 |
| rHV2-Lys 47 | 1,8 | 3,0 | 16 | 30 |
| TT : temps de thrombine SC : substrat chromogène | | | | |

d) Les concentrations plasmatiques des deux variants d'hirudine obtenues après 30 minutes de perfusion continue i.v. chez le lapin sont en relation linéaire avec les doses perfusées.

e) Effets antithrombotiques

| PRODUITS | LAPIN i.v. perfusion ug/kg/h | | RAT i.v. bolus ug/kg | |
|---|---|---|---|---|
| | WESSLER | STASE | WESSLER | |
| | | | -5min 40s | -1min |
| rHV2 | 375 | -- | 240 | - |
| rHV2-Lys 47 | 20 | 47 | 160 | - |
| Héparine Standard | 110 | 110 | 160 | 110 |

Sur la base de ces résultats pharmacologiques préliminaires il apparaît qu'en perfusion continue chez le lapin, le variant d'hirudine recombinante rHV2-Lys 47 a une acvtité antithrombotique supérieure à celle de rHV2 et de l'héparine. La pharmacocinétique des deux variants d'hirudine est identique.

Exemple 6 Comparaison du rapport activité antithrombotique/risque hémorragique de l'hirudine rHV2-Lys 47 et l'héparine standard.

Les études réalisées montrent que le risque hémorragique chez le lapin ou l'allongement du temps de saignement chez le rat, traités au rHV2-Lys 47, sont inférieurs à ceux traités à l'héparine standard en se référant, pour chaque espèce, à des doses équiactives dans un modèle de thrombose.

Dépôt de souches représentatives de l'invention

Les souches suivantes ont été déposées à la Collection Nationale de Cultures de Microorganismes de l'Institut Pasteur, 28 Rue du Docteur Roux, Paris (15), le 6 novembre 1986 :
TGY1sp4 pTG1977 (HV2-Arg$^{47}$) sous le n° I-621
TGY1sp4 pTG1980 (HV2-Lys$^{47}$) sous le n° I-622.
La souche de référence avait été déposée le 6 juin 1986 :
TGY1sp4 pTG1828 sous le n° I-569.

**Revendications**
**Revendications pour les Etats contractants suivants : AT, BE, CH, DE, FR, GB, IT, LI, LU, NL, SE**

1. Un variant hirudine sélectionné parmi un variant ayant une séquence d'acides amines substantiellement de type HV2 dans laquelle le résidu Asn en position 47 a été remplacé par un résidu Lys, Arg ou His et un variant ayant une séquence d'acides aminés substantiellement de type HV1, HV2 ou HV3 dans laquelle le résidu Tyr en position 63 a été remplacé par un résidu Glu ou Asp.

2. Un variant hidurine selon la revendication 1, ayant une séquence d'acides aminés substantiellement de type HV2 dans laquelle le résidu Asn en position 47 a été remplacé par un résidu Lys, Arg ou His.

3. Un variant hirudine selon la revendication 2, ayant une séquence d'acides amines de type HV2 dans laquelle le résidu Asn en position 47 a été remplacé par un résidu Lys.

4. Un variant hirudine selon la revendication 1, ayant une séquence d'acides aminés substantiellement de type HV1, HV2 ou HV3 dans laquelle le résidu Tyr en position 63 a été remplacé par un résidu Glu ou Asp.

5. Un variant hidurine selon la revendication 4, ayant une séquence d'acides amines substantiellement de type HV2 dans laquelle le résidu Tyr en position 63 a été remplacé par un résidu Glu ou Asp.

6. Un variant de l'hirudine ayant la séquence suivante en amino acides :

```
ILE THR TYR THR ASP CYS THR GLU SER GLY GLN ASN LEU CYS LEU
CYS GLU GLY SER ASN VAL CYS GLY LYS GLY ASN LYS CYS ILE LEU
GLY SER ASN GLY LYS GLY ASN GLN CYS VAL THR GLY GLU GLY THR
PRO LYS PRO GLU SER HIS ASN ASN GLY ASP PHE GLU GLU ILE PRO
GLU GLU TYR LEU GLN
```

**7.** A titre d'inhibiteur de la thrombine, un variant hirudine selon l'une des revendications 1 à 6.

**8.** L'usage thérapeutique d'un variant selon l'une des revendications 1 à 6.

**9.** Une composition pharmaceutique anti-coagulante qui comprend à titre de principe actif, un variant selon l'une des revendications 1 à 6.

**10.** Un procédé de fabrication d'un variant selon l'une des revendications 1 à 6, qui comprend l'acte de cultiver une cellule transformée par un fragment d'ADN codant pour un variant selon l'une des revendications 1 à 6 et capable d'exprimer ledit fragment d'ADN et l'acte de récolter ledit variant à partir de la culture.

**11.** Un procédé selon la revendication 11, caractérisé en ce qu'il comporte la fermentation d'une levure préalablement transformée soit par un plasmide comportant :
- l'origine de réplication du plasmide de levure 2 μ,
- le gène ura3,
- une origine de réplication dans E. Coli et un marqueur de résistance à un antibiotique,
- un promoteur de transcription, la séquence "leader" et la séquence pre-pro du précurseur du facteur alpha, fusionnée en phase, en amont de la séquence codant du variant de l'hirudine,
- le terminateur de transcription du gène PCK de la levure qui sera placé en aval du gène dudit variant,

soit par un bloc fonctionnel d'ADN codant pour ledit variant de l'hirudine.

**Revendications pour les Etats contractants suivants : ES, GR**

**1.** Procédé de fabrication d'un variant de l'hirudine sélectionné parmi un variant ayant une séquence d'acides aminés substantiellement de type HV2 dans laquelle le résidu Asn en position 47 a été remplacé par un résidu Lys, Arg ou His et un variant ayant une séquence d'acides aminés substantiellement de type HV1, HV2 ou HV3 dans laquelle le résidu Tyr en position 63 a été remplacé par un résidu Glu ou Asp, dans lequel ledit variant est obtenu par un procédé comportant des étapes de synthèse, d'hémisynthèse et/ou de manipulation génétique.

**2.** Procédé de fabrication d'un variant de l'hirudine selon la revendication 1, caractérise en ce qu'il comprend :
(i) la culture d'une cellule qui est transformée par un fragment d'ADN hétérologue codant pour un desdits variants et qui est capable d'exprimer ledit fragment d'ADN et
(ii) la récupération dudit variant à partir de la culture.

**3.** Procédé de fabrication d'un variant de l'hirudine selon la revendication 1, caractérise en ce qu'il comporte la fermentation d'une levure préalablement transformée soit par un plasmide comportant :
- l'origine de réplication du plasmide de levure 2 μ,
- le gène ura3,
- une origine de réplication dans E. Coli et un marqueur de résistance à un antibiotique,
- un promoteur de transcription, la séquence "leader" et la séquence pre-pro du précurseur du facteur alpha, fusionnée en phase, en amont de la séquence codant du variant de l'hirudine,
- le terminateur de transcription du gène PCK de la levure qui sera placé en aval du gène dudit variant,

soit par un bloc fonctionnel d'ADN codant pour ledit variant de l'hirudine.

**4.** Procédé de fabrication d'un variant de l'hirudine selon l'une des revendications 1 à 3, caractérisé en ce que le variant de l'hirudine a la séquence suivante en amino acides :

```
ILE THR TYR THR ASP CYS THR GLU SER GLY GLN ASN LEU CYS LEU
CYS GLU GLY SER ASN VAL CYS GLY LYS GLY ASN LYS CYS ILE LEU
GLY SER ASN GLY LYS GLY ASN GLN CYS VAL THR GLY GLU GLY THR
PRO LYS PRO GLU SER HIS ASN ASN GLY ASP PHE GLU GLU ILE PRO
GLU GLU TYR LEU GLN
```

## Claims

Claims for the following Contracting States : AT, BE, CH, DE, FR, GB, IT, LI, LU, NL, SE

**1.** A variant of hirudine chosen among a variant having a sequence of amino acids substantially of the HV2 type in which the residue Asn at position 47 is replaced by the residue Lys, Arg or His and a variant having a sequence of amino acids substantially of the HV1, HV2 or HV3 type, in which the residue Tyr at position 63 is replaced by a residue Glu or Asp.

**2.** The variant of hirudine as claimed in claim 1, having a sequence of amino acids substantially of the HV2 type in which the residue Asn at position 47 is replaced by the residue Lys, Arg or His.

**3.** The variant of hirudine as claimed in claim 2, having a sequence of amino acids of the HV2 type in which the residue Asn at position 47 is replaced by a residue Lys.

**4.** The variant of hirudine as claimed in claim 1, having a sequence of amino acids substantially of the HV1, HV2 or HV3 type, in which the residue Tyr at position 63 is replaced by a residue Glu or Asp.

**5.** The variant of hirudine as claimed in claim 4, having a sequence of amino acids substantially of the HV2 type in which the residue Tyr at position 63 is replaced by a Glu or Asp.

**6.** The variant of hirudine having the following sequence of amino acids :

```
ILE THR TYR THR ASP CYS THR GLU SER GLY GLN ASN LEU CYS LEU
CYS GLU GLY SER ASN VAL CYS GLY LYS GLY ASN LYS CYS ILE LEU
GLY SER ASN GLY LYS GLY ASN GLN CYS VAL THR GLY GLU GLY THR
PRO LYS PRO GLU SER HIS ASN ASN GLY ASP PHE GLU GLU ILE PRO
GLU GLU TYR LEU GLN
```

**7.** By way of a thrombine inhibitor, a variant of hirudine as claimed in one of claims 1 to 6.

**8.** Therapeutic use of a variant as claimed in one of claims 1 to 6.

**9.** By way of an anticoagulant agent, a composition containing, by way of active principle, at least one variant as claimed in one of claims 1 to 6.

**10.** A method of manufacturing of a variant as claimed in one of claims 1 to 6, comprising the action of cultivating a transformed cell with a DNA fragment coding for a variant as claimed in one of claims 1 to 6, and able to express said DNA fragment and the action of collecting said variant from the culture.

**11.** The method as claimed in claim 10, characterised that comprises the fermentation of a yeast previously transformed either with a plasmid containing:
- the origin of replication of the 2$\mu$ plasmid of yeast,
- the ura3 gene,
- an origin of replication in E. coli and a marker for resistance to an antibiotic,
- a transcription promoter, the "Leader" sequence and the prepro sequence of the precursor of the alpha factor, this sequence being used in phase, upstream from the coding sequence for the variant of hirudine,
- the transcription terminator of the PGK gene of yeast, which will be placed downstream from the gene for the said variant,

or by a functional DNA block coding for the said variant of hirudin.

**Claims for the following Contracting States : ES, GR**

1. Method for manufacturing a variant of hirudine chosen among a variant having a sequence of amino acid substantially of the HV2 type in which the residue Asn at position 47 is replaced by the residue Lys, Arg or His and a variant having a sequence of amino acids substantially of the HV1, HV2 or HV3 type, in which the residue Tyr at position 63 is replaced by a residue Glu or Asp, in which said variant is obtained with a method comprising steps of synthesis, hemisynthesis and/or genetical manipulation.

2. The method of manufacturing a variant of hirudine as claimed in claim 1, characterised that it comprises:
   (i) cultivating a cell which is transformed with heterologous DNA fragment coding for one of said variants and which is able to express DNA fragment and,
   (ii) collecting said variant from the culture.

3. The method as claimed in claim 1, characterised in that it comprises the fermentation of a yeast previously transformed either with a plasmid containing:
   - the origin of replication of the 2$\mu$ plasmid of yeast,
   - the ura3 gene,
   - an origin of replication in E. coli and a marker for resistance to an antibiotic,
   - a transcription promoter, the "Leader" sequence and the prepro sequence of the precursor of the alpha factor, this sequence being used in phase, upstream from the coding sequence for the variant of hirudine,
   - the transcription terminator of the PGK gene of yeast, which will be placed downstream from the gene for the said variant,
   or by a functional DNA block coding for the said variant of hirudine.

4. The method of manufacturing a variant of hirudine as claimed in one of claims 1 to 3, characterised in that the variant of hirudine has the following sequence of amino acids :

**ILE THR TYR THR ASP CYS THR GLU SER GLY GLN ASN LEU CYS LEU**

**CYS GLU GLY SER ASN VAL CYS GLY LYS GLY ASN LYS CYS ILE LEU**

**GLY SER ASN GLY LYS GLY ASN GLN CYS VAL THR GLY GLU GLY THR**

**PRO LYS PRO GLU SER HIS ASN ASN GLY ASP PHE GLU GLU ILE PRO**

**GLU GLU TYR LEU GLN**

**Patentansprüche**
**Patentansprüche für folgenden Vertragsstaaten : AT, BE, CH, DE, FR, GB, IT, LI, LU, NL, SE**

1. Hirudin-Variante, selektiert aus einer Variante mit einer Aminosäuresequenz im wesentlichen vom HV2-Typ, in welcher der Rest Asn in der Position 47 durch einen Rest Lys, Arg oder His ersetzt worden ist, und einer Variante mit einer Aminosäuresequenz im wesentlichen vom HV1-, HV2- oder HV3-Typ, in dem der Rest Tyr in der Position 63 durch einen Rest Glu oder Asp ersetzt worden ist.

2. Hirudin-Variante nach Anspruch 1, die eine Aminosäuresequenz im wesentlichen vom HV2-Typ aufweist, in welcher der Rest Asn in der Position 47 durch einen Rest Lys, Arg oder His ersetzt worden ist.

3. Hirudin-Variante nach Anspruch 2, die eine Aminosäuresequenz vom HV2-Typ aufweist, in welcher der Rest Asn in der Position 47 durch einen Rest Lys ersetzt worden ist.

4. Hirudin-Variante nach Anspruch 1, die eine Aminosäuresequenz im wesentlichen vom HV1-, HV2- oder HV3-Typ aufweist, in welcher der Rest Tyr in der Position 63 durch einen Rest Glu oder Asp ersetzt worden ist.

5. Hirudin-Variante nach Anspruch 4, die eine Aminosäuresequenz im wesentlichen vom HV2-Typ aufweist, in welcher der Rest Tyr in der Positon 63 durch einen Rest Glu oder Asp ersetzt worden ist.

6. Hirudin-Variante, welche die nachstehend angegebene Sequenz an Aminosäuren aufweist:

```
ILE THR TYR THR ASP CYS THR GLU SER GLY GLN ASN LEU CYS LEU
CYS GLU GLY SER ASN VAL CYS GLY LYS GLY ASN LYS CYS ILE LEU
GLY SER ASN GLY LYS GLY ASN GLN CYS VAL THR GLY GLU GLY THR
PRO LYS PRO GLU SER HIS ASN ASN GLY ASP PHE GLU GLU ILE PRO
GLU GLU TYR LEU GLN .
```

7. Hirudin-Variante nach einem der Ansprüche 1 bis 6 als Thrombin-Inhibitor.

8. Therapeutische Verwendung einer Variante nach einem der Ansprüche 1 bis 6.

9. Pharmazeutische Antikoagulans-Zusammensetzung, die als aktives Prinzip (Wirkstoff) eine Variante nach einem der Ansprüche 1 bis 6 enthält.

10. Verfahren zur Herstellung einer Variante nach einem der Ansprüche 1 bis 6, das umfaßt die Kultivierung einer Zelle, die durch ein ADN-Fragment transformiert worden ist, das für eine Variante nach einem der Ansprüche 1 bis 6 codiert und dieses ADN-Fragment exprimieren kann, und das Ernten (Gewinnen) dieser Variante aus der Kultur.

11. Verfahren nach Anspruch 10, dadurch gekennzeichnet, daß es umfaßt die Fermentation eines Hefepilzes, der vorher transformiert worden ist,
entweder durch ein Plasmid, das aufweist:
   - den Replikationsursprung des Hefepilz-Plasmids 2 μ,
   - das Gen ura3,
   - einen Replikationsursprung in E. coli und einen Resistenz-Marker gegenüber einem Antibiotikum,
   - einen Transkriptionspromotor, wobei die "leader"-Sequenz und die pre-pro-Sequenz des Vorläufers des α-Faktors in Phase fusioniert sind stromaufwärts von der Sequenz, die für die Hirudin-Variante codiert,
   - den Transkriptions-Terminator des Gens PCK des Hefepilzes, der stromabwärts von dem Gen dieser Variante angeordnet ist,
oder durch einen funktionellen ADN-Block, der für die genannte Hirudin-Variante codiert.

**Patentansprüche für folgenden Vertragsstaaten : ES, GR**

1. Verfahren zur Herstellung einer Hirudin-Variante, selektiert aus einer Variante mit einer Aminosäuresequenz im wesentlichen vom HV2-Typ, in welcher der Rest Asn in der Position 47 durch einen Rest Lys, Arg oder His ersetzt worden ist, und einer Variante mit einer Aminosäuresequenz im wesentlichen vom HV1-, HV2- oder HV3-Typ, in welcher der Rest Tyr in der Position 63 durch einen Rest Glu oder Asp ersetzt worden ist, bei dem die Variante nach einem Verfahren erhalten wird, das Synthese-, Halbsynthese- und/oder Genmanipulations-Stufen umfaßt.

2. Verfahren zur Herstellung einer Hirudin-Variante nach Anspruch 1, dadurch gekennzeichnet, daß es umfaßt:

(i) die Kultivierung einer Zelle, die durch ein heterologes ADN-Fragment transformiert worden ist, das für eine dieser Varianten codiert und das ADN-Fragment: exprimieren kann, und

(ii) die Gewinnung (Abtrennung) dar Variante von der Kultur.

3. Verfahren zur Herstellung einer Hirudin-Variante nach Anspruch 1, dadurch gekennzeichnet, daß es unfaßt die Fermentation eines Hefepilzes, der vorher transformiert worden ist

entweder durch ein Plasmid, das enthält:
- den Replikationsursprung des Hefepilz-Plasmids 2μ,
- das Gen ura3,
- einen Replikationsursprung in E. coli und einen Resistenz-Marker gegenüber einem Antibiotikum,
- einen Transkriptionspromotor, wobei die "leader"-Sequenz und die pre-pro-Sequenz des Vorläufers des α-Faktors in Phase fusioniert wurden stromaufwärts der Sequenz, die für die Hirudin-Variante codiert,
- den Transkriptions-Terminator des Gens PCK des Hefepilzes, der stromabwärts des Gens dieser Variante angeordnet ist,

oder durch einen funktionellen ADN-Block, der für die genannte Hirudin-Variante codiert.

4. Verfahren zur Herstellung einer Hirudin-Variante nach einem der Ansprüche 1 bis 3, dadurch gekennzeichnet, daß die Hirudin-Variante die folgende Sequenz an Aminosäuren aufweist:

ILE THR THR THR ASP CYS THR GLU SER GLY GLN ASN LEU CYS LEU

CYS GLU GLY SER ASN VAL CYS GLY LYS GLY ASN LYS CYS ILE LEU

GLY SER ASN GLY LYS GLY ASN GLN CYS VAL THR GLY GLU GLY THR

PRO LYS PRO GLU SER HIS ASN ASN GLY ASP PHE GLU GLU ILE PRO

GLU GLU THR LEU GLN

## FIG.1

| | 1 | 2 | 3 | 4 | 5 | 6 | 7 | 8 | 9 | 10 | 11 | 12 | 13 | 14 | 15 |
|---|---|---|---|---|---|---|---|---|---|---|---|---|---|---|---|
| 1 HV1 | VAL | VAL | TYR | THR | ASP | CYS | THR | GLU | SER | GLY | GLN | ASN | LEU | CYS | LEU |
| 2 HV2 | ILE | THR | TYR | THR | ASP | CYS | THR | GLU | SER | GLY | GLN | ASN | LEU | CYS | LEU |
| 3 HV3 | ILE | THR | TYR | THR | ASP | CYS | THR | GLU | SER | GLY | GLN | ASN | LEU | CYS | LEU |

| 16 | 17 | 18 | 19 | 20 | 21 | 22 | 23 | 24 | 25 | 26 | 27 | 28 | 29 | 30 |
|---|---|---|---|---|---|---|---|---|---|---|---|---|---|---|
| CYS | GLU | GLY | SER | ASN | VAL | CYS | GLY | CLN | GLY | ASN | LYS | CYS | ILE | LEU |
| CYS | GLU | GLY | SER | ASN | VAL | CYS | GLY | LYS | GLY | ASN | LYS | CYS | ILE | LEU |
| CYS | GLU | GLY | SER | ASN | VAL | CYS | GLY | LYS | GLY | ASN | LYS | CYS | ILE | LEU |

| 31 | 32 | 33 | 34 | 35 | 36 | 37 | 38 | 39 | 40 | 41 | 42 | 43 | 44 | 45 | 46 | 47 |
|---|---|---|---|---|---|---|---|---|---|---|---|---|---|---|---|---|
| GLY | SER | ASP | GLY | CLU | LYS | ASN | GLN | CYS | VAL | THR | GLY | GLU | GLY | THR | PRO | LYS |
| GLY | SER | ASN | GLY | LYS | GLY | ASN | GLN | CYS | VAL | THR | GLY | GLU | GLY | THR | PRO | ASN |
| GLY | SER | CLN | GLY | LYS | ASP | ASN | GLN | CYS | VAL | THR | GLY | GLU | GLY | THR | PRO | LYS |

| 48 | 49 | 50 | 51 | 52 | 53 | 54 | 55 | 56 | 57 | 58 | 59 | 60 | 61 | 62 | 63 | 64 | 63 |
|---|---|---|---|---|---|---|---|---|---|---|---|---|---|---|---|---|---|
| PRO | GLX | SER | HIS | ASN | ASP | GLY | ASP | PHE | GLU | GLU | ILX | PRO | GLU | CLU | TYR | LEU | CLN |
| PRO | GLU | SER | HIS | ASN | ASN | GLY | ASP | PHE | GLU | GLU | ILX | PRO | GLU | CLU | TYR | LEU | CLN |
| PRO | GLN | SER | HIS | ASN | GLN | GLY | ASP | PHE | GLU | PRO | ILX | PRO | GLU | ASP | TYR | ASP | GLU |

63 → 64
↓
ALA 63

64  65  66

1. d'après DODT et al. FEBS LETTERS 1984 _165_, 180-183.
2. d'après HARVEY et al. Proc. Natl. Acad. USA 1986 _83_, 1084-1088
3. d'après DODT et al. Biol. Chem. Hoppe-Seyler 1986 367, 803-811.

● pTG1828
■ pTG1980
○ pTG1977
□ pTG1978
▲ pTG1979
△ pTG881

FIG.2